# EUROPÄISCHE PATENTSCHRIFT

(11) **EP 3 130 305 B1**
(45) Veröffentlichungstag und Bekanntmachung des Hinweises auf die Patenterteilung: **13.01.2021**
(21) Anmeldenummer: 15180826.8
(22) Anmeldetag: 12.08.2015
(51) Int. Cl.: A61B 90/50, F16M 13/02, B25J 1/02, A61B 90/57

(54) **MEDIZINISCHER HALTEARM**
MEDICAL HOLDING ARM
BRAS DE SUPPORT MEDICAL

(43) Veröffentlichungstag der Anmeldung: 15.02.2017
(60) Teilanmeldung: 17184477.2
(73) Patentinhaber: medineering GmbH, 81829 München (DE)
(72) Erfinder: NOWATSCHIN, Stephan, 81677 München (DE); KRINNINGER, Maximilian, 82234 Weßling-Oberpfaffenhofen (DE); GIERLACH, Dominikus, 80798 München (DE)
(74) Vertreter: Eisenführ Speiser

(56) Entgegenhaltungen:
- EP-A1- 0 855 002
- EP-A2- 2 965 874
- EP-B1- 1 958 587
- WO-A1-2016/075241
- WO-A2-2007/005555
- CN-U- 203 622 451
- DE-A1-102009 009 549
- DE-A1-102011 004 371
- JP-A- 2012 218 139
- JP-A- 2013 006 239
- JP-A- 2014 008 071
- JP-B2- 3 183 355
- US-A1- 2003 167 061
- US-A1- 2013 221 183

## Beschreibung

Die Erfindung betrifft eine Haltevorrichtung, insbesondere Haltearm und/oder Stativ, für medizinische Zwecke, insbesondere zum Halten von chirurgischen mechatronischen Assistenzsystemen und/oder chirurgischen Instrumenten. Weiterhin betrifft die Erfindung ein Verfahren.

Haltearme, die unter Haltevorrichtungen der eingangs genannten Art fallen, sind bereits seit längerem bekannt und werden in der Chirurgie insbesondere dazu eingesetzt, einen Operateur von statischer Haltearbeit zu entlasten. Ein derartiger Haltearm wird eingesetzt, um ein mechatronisches Assistenzsystem und/oder ein chirurgisches Instrument zu halten, wie etwa ein Manipulator, ein Endoskop, eine chirurgische Klemme und dergleichen. Insbesondere zum Halten von Endoskopen haben sich die eingangs genannten Haltearme bewährt. Bei der endoskopischen Chirurgie bedient ein Operateur in der Regel mit beiden Händen ein Instrument, während ein Assistent das Endoskop hält, um das Operationsfeld über einen Bildschirm sichtbar zu machen. Das Halten des Endoskops über einen längeren Zeitraum ist sehr ermüdend. Aus diesem Grund werden vermehrt vorgenannte Haltearme eingesetzt.

Ein solcher Haltearm ist beispielsweise aus DE 195 26 915 B4 bekannt. Die dort offenbarte Haltevorrichtung für medizinische Zwecke weist ein Anschlussteil und einen Halter für chirurgische Werkzeuge sowie einen zwischen dem Halter und dem Anschlussteil angeordneten Arm auf. Der Arm ist mit dem Halter und dem Anschlussteil oder mit einem benachbarten Arm über ein Gelenk verbunden und mit einer pneumatisch betätigbaren Vorrichtung zur wahlweisen Festlegung und Lösung der Gelenke überkoppelt, wobei diese Vorrichtung die Gelenke unter Einwirkung einer Bremskraft auf das Gelenk ausübenden mechanischen Feder festlegt und wobei die Vorrichtung gegen die Kraft dieser Feder pneumatisch in einen das Gelenk freigebenden Zustand überführbar ist. An dem proximalen Ende des Arms ist ein Betätigungsorgan angeordnet, mittels dessen Hilfe ein Ventil öffenbar ist, sodass die einzelnen Gelenke des Arms verstellt werden können. Bei Loslassen des Betätigungsorgans wird das Ventil wieder geschlossen, sodass die Gelenke festgelegt sind. Nachteilig ist, dass mit dem Haltearm alle Gelenke gleichzeitig geöffnet werden, wodurch eine Positionierung schwierig sein kann.

Ein ähnlicher Haltearm ist in EP 1 958 587 B1 offenbart. Der dort offenbarte Haltearm weist ebenfalls mehrere Gelenke auf, und zur Betätigung der Gelenke ist ein berührungssensitiver Sensor vorgesehen. Der Sensor ist am Haltearm benachbart zum medizinischen Instrument angeordnet, sodass bei Ergreifen des medizinischen Instruments der Operator in Kontakt kommt mit dem berührungssensitiven Sensor, wodurch alle Gelenke des Haltearms freigegeben werden. Auch hier tritt das oben genannte Problem der mangelhaften Positionierung auf.

DE 10 2011 004 371 A1 offenbart einen Haltearm zur Halterung von chirurgischen Instrumenten. Dieser umfasst mindestens ein steuerbares Gelenk, das in einen beweglichen Zustand und in einen arretierten Zustand schaltbar ist. Eine steuerbare Klemmvorrichtung ist in einen geöffneten Zustand und in einen geklemmten Zustand schaltbar um ein chirurgisches Instrument zu greifen. Mittels einer Steuereinheit können Fernsteuerbefehle empfangen und diese in Schaltvorgänge des Gelenks und/oder der Klemmvorrichtung umgesetzt werden. In einer Anordnung mit einem Operationstisch ist vorzugsweise eine Fernbedieneinheit umfasst.

Weiterhin besteht bei beiden oben genannten Haltearmen das Problem, dass der Bediener darüber im Unklaren ist, ob tatsächlich alle Gelenke geöffnet sind, wie weit diese geöffnet sind, und welche Bewegungen zulässig sind.

Aus EP 2 455 053 B1 ist ferner ein Tragesystem zum Tragen bzw. zum Stützen medizintechnischer Geräte in einem Behandlungsraum bekannt. Das Tragesystem weist ein Stativ zum Montieren im Behandlungsraum, mindestens ein Gelenk bzw. mindestens einen Mechanismus, mit dem das Stativ im Behandlungsraum bewegbar ist, eine Bedienungshilfe zur Betätigung mindestens eines Teils des Tragesystems, um eine Bewegung des Stativs zu steuern und ein Rückmeldesystem zur Erzeugung eines Signals bzw. einer Rückmeldung über eine Betätigung des korrelierenden Teils des Tragesystems mit der Bedienungshilfe auf. Das Tragesystem zeichnet sich dadurch aus, dass das Rückmeldesystem mindestens einen Leuchtkörper aufweist, der den Teil des Tragesystems bzw. das entsprechende Gelenk oder den entsprechenden Mechanismus örtlich am Stativ an der jeweiligen Stelle des Mechanismus bzw. am entsprechenden Gelenk identifiziert bzw. kennzeichnet, typischerweise durch Beleuchtung. In dem gezeigten Ausführungsbeispiel umfasst das Tragesystem zwei Arme, die um parallel zueinander angeordnete Schwenkachsen verschwenkbar sind. An jedem Gelenk ist ein kurzes zylindrisches Stück vorgesehen, und in diesem zylindrischen Stück ist eine oval förmige Lampe angeordnet. Die Bedieneinheit ist als Fernbedienung ausgebildet und weist Tasten auf zum Freigeben jedes Gelenks. Die entsprechende Lampe an dem entsprechenden Gelenk leuchtet dann auf, wenn die Taste gedrückt ist. Dadurch soll es insbesondere in dunklen OP-Räumen einfacher sein, die richtige Taste zu drücken und so das richtige Gelenk freizugeben bzw. hierüber Feedback zu erhalten.

Nachteilig ist hieran, dass allein dann eine Lampe aufleuchtet, wenn der entsprechende Knopf an der Fernbedienung gedrückt ist, also das Gelenk betätigt ist.

Schließlich offenbart EP 0 855 002 A1 einen Haltearm, bei dem Kräfte oder Momente, die sich aus einer Unbalance ergeben gemessen werden, um eine gezielte Verstellung von Ausgleichsgewichten vornehmen zu können.

Aufgabe der vorliegenden Erfindung ist es daher, die Sicherheit der Bedienung einer derartigen Haltevorrichtung, sowie deren Bedienfreundlichkeit zu verbessern.

Die Erfindung löst die Aufgabe bei einer Haltevorrichtung der eingangs genannten Art mit einem proximalen Ende zum Befestigen der Haltevorrichtung an einer Basis und einem distalen Ende zum Aufnehmen eines Anbaugeräts, wenigstens einem ersten und einem zweiten Armsegment, wobei das erste Armsegment mit einem ersten Gelenk und das zweite Armsegment mit einem zweiten Gelenk verbunden ist, wobei jedes Gelenk freigebbar und arretierbar ist, einer Bedieneinrichtung zum Freigeben und/oder Arretieren des entsprechenden Gelenks zum Verbringen der Haltevorrichtung in eine gewünschte Pose, und einer ersten Anzeigeeinheit, die an dem ersten Gelenk angeordnet ist und einer zweiten

Anzeigeeinheit, die an dem zweiten Gelenk angeordnet ist, wobei die erste und/oder zweite Anzeigeeinheit dazu eingerichtet sind, wenigstens einen vom Freigeben und Arretieren des entsprechenden Gelenks verschiedenen Status der Haltevorrichtung und/oder eines Anbaugeräts anzuzeigen. Das Anzeigen erfolgt bevorzugt mittels Anzeigen einer den Status repräsentierenden Repräsentation. Der Erfindung liegt die Erkenntnis zugrunde, dass allein das Anzeigen des Freigebens oder Arretierens eines entsprechenden Gelenks, also der Betätigung, unter Umständen nicht ausreicht, um eine ausreichende Sicherheit und/oder Bedienfreundlichkeit zu erreichen. Das Anzeigen des wenigstens einen vom Freigeben und Arretieren des entsprechenden Gelenks verschiedenen Status der Haltevorrichtung und/oder eines Anbaugeräts kann dabei zusätzlich oder alternativ zum Anzeigen des freigegebenen oder arretierten Status der Haltevorrichtung erfolgen. Die Anzeige stellt dem Bediener dadurch weitere, über den reinen Status des Freigebens und Arretierens eines Gelenks hinausgehende Kennzeichnungen bereit, durch die die Bedienfreundlichkeit bzw. Sicherheit dann erhöht ist. Die Anzeigeeinheit kann elektrisch, mechanisch oder elektronisch ausgebildet sein und mittels visuellen und/oder akustischen Anzeigen arbeiten.

Gemäß einer ersten bevorzugten Ausführungsform weisen die Anzeigeeinheiten jeweils wenigstens eine Lichtquelle auf. In einer solchen Ausführungsform ist die den Status repräsentierende Repräsentation das Aufleuchten der Lichtquelle(n). Dies ist eine besonders einfache Art, die Anzeigeeinheiten auszubilden. Ferner ist diese visuell erfassbare Anzeigeeinheit auch bei schlechten Sichtverhältnissen erfassbar. Vorzugsweise ist die Lichtquelle dazu eingerichtet, in zwei oder mehr verschiedenen Farben aufzuleuchten und dadurch kann verschiedenen Status eine verschiedene Farbe zugeordnet werden, wodurch weiterhin Sicherheit und Bedienfreundlichkeit verbessert sind.

In einer bevorzugten Weiterbildung weisen die Anzeigeeinheiten wenigstens ein Display zum Anzeigen des Status auf. Bevorzugt weist wenigstens eine der Anzeigeeinheiten ein derartiges Display auf. Besonders bevorzugt weist jede der Anzeigeeinheiten ein Display zum Anzeigen des Status auf. Ein solches Display kann beispielsweise als berührungsempfindliches Display ausgebildet sein und zeigt bevorzugt sowohl graphische, als auch alphanummerische Wiedergaben an. So ist es denkbar bevorzugt, dass beispielsweise Patientendaten, wie Alter, Gewicht, Erkrankungen und dergleichen angezeigt werden, als auch Daten aus einer Operation, wie beispielsweise Körperfunktionsdaten oder Anästhesiedaten. Auch ist es denkbar in einem solchen Fall, dass die Anzeigeeinheiten nicht ausschließlich dazu dienen, den Status der Haltevorrichtung anzuzeigen, sondern auch einen Status eines Patienten und/oder einer Umgebung, oder zum Anzeigen von Informationen.

Gemäß einer weiteren bevorzugten Ausführungsform ist wenigstens eine Anzeigeeinheit im Wesentlichen ringförmig um eine Schwenkachse des entsprechenden Gelenks ausgebildet. Beispielsweise ist die Anzeigeeinheit als ringförmige Lichtquelle, beispielsweise ein Ring aus LED-Elementen, als ringförmiges OLED, als ringförmiges Display oder dergleichen ausgebildet. Ein LED-Ring kann dabei beispielsweise einreihig oder zwei- oder mehrreihig ausgebildet sein. Bevorzugt ist die im Wesentlichen ringförmige Anzeigeeinheit koaxial zur Schwenkachse des entsprechenden Gelenks ausgebildet. Dadurch ist die Anzeigeeinheit von jeder Seite der Schwenkachse aus sichtbar und gleichzeitig ein Indikator für den Verlauf der Schwenkachse. Bevorzugt sind wenigstens zwei derartige Anzeigeeinheiten an einem Gelenk angeordnet und zwar so, dass sie beide koaxial zur Schwenkachse angeordnet sind.

Gemäß einer bevorzugten Weiterbildung ist der Status ein Betriebsbereitheits-Status der Haltevorrichtung. Beispielsweise ist die Anzeigeeinheit dazu eingerichtet, in einer ersten Farbe zu leuchten, wenn die Haltevorrichtung betriebsbereit ist, und in einer zweiten Farbe zu leuchten, wenn sie nicht betriebsbereit ist. Vorzugsweise leuchten alle Anzeigevorrichtungen gleichzeitig und/oder blinken auf, um dies anzuzeigen. So ist es denkbar und bevorzugt, dass nach einem Einschalten der Haltevorrichtung, zunächst ein interner Mikroprozessor die Betriebsbereitheit überprüft, und in Abhängigkeit davon der Betriebsbereitheits-Status mittels der Anzeigeeinheiten angezeigt wird. Hierdurch kann ein Bediener sofort erkennen, ob die Haltevorrichtung betriebsbereit ist und beispielsweise in einer chirurgischen Operation eingesetzt werden kann, oder ob ein Defekt oder dergleichen vorliegt.

In einer weiteren bevorzugten Ausführungsform ist der Status ein Abstand zwischen einem Anbaugerät und einem Rand eines vordefinierten Arbeitsbereichs der Haltevorrichtung. Durch Lagesensoren in den Gelenken oder dergleichen kann eine Lage der Haltevorrichtung bestimmt werden, und so ein Abstand zwischen einem Anbaugerät, welches an der Haltevorrichtung aufgenommen ist, oder im Abschnitt der Haltevorrichtung selbst, und einem Rand eines vordefinierten Arbeitsbereichs, bestimmt werden. Bevorzugt ist vorgesehen, dass die Anzeigevorrichtungen den Abstand visuell anzeigen. Beispielsweise ist bevorzugt, dass die Anzeigevorrichtungen ihre Leuchtintensität ändern, beispielsweise erhöhen, um den Abstand anzuzeigen, von einer ersten in eine zweite Farbe wechseln, und/oder einen Warnton ausgeben. Beispielsweise kann vorgesehen sein, dass die Anzeigevorrichtungen diskret in zwei Farben leuchten, und der Anteil der einen Farbe reduziert wird, wenn die Haltevorrichtung auf den Rand zu bewegt wird.

Bevorzugt ist ferner vorgesehen, dass der Status ein Abstand zwischen einer aktuellen Pose der Haltevorrichtung und einer vordefinierten Pose der Haltevorrichtung ist. Hierdurch wird ein Operateur dabei unterstützt, den Haltearm in die korrekte Pose zu bewegen. Beispielsweise kann vorgesehen sein, dass einige oder alle Anzeigevorrichtungen orange leuchten, wenn die Pose noch nicht erreicht ist, und nach und nach in eine grüne Farbe wechseln, wenn das entsprechende Gelenk die entsprechende vordefinierte Pose erreicht hat. Hierdurch ist sowohl die Sicherheit als auch die Bedienfreundlichkeit wesentlich verbessert, und ein Operateur erhält unmittelbar und direkt Feedback darüber, ob die Haltevorrichtung in der korrekten Pose ist.

Dabei, oder bei einer anderen Ausführungsform, kann zusätzlich vorgesehen sein, dass die Anzeigeeinheiten dazu eingerichtet sind, eine Richtung anzuzeigen, in die wenigstens ein Gelenk zu bewegen ist, um die Haltevorrichtung von der aktuellen Pose in die vordefinierte Pose zu bewegen. Dies erfolgt vorzugsweise mittels Blinken, Rotieren eines Musters, Variation der Helligkeit, Variation der Farbe. Vorzugsweise werden einige oder alle Gelenke bei Erreichen der vordefinierten Pose automatisch arretiert. Eine derartige vordefinierte Pose kann beispielsweise über ein OP-System oder über eine OP-Planungssoftware erhalten werden.

In einer weiteren bevorzugten Ausführungsform ist der Status ein Arbeitsstatus einer Steuerung der Haltevorrichtung. Die Haltevorrichtung weist bevorzugt eine Steuerung, beispielsweise einen Mikrocontroller oder dergleichen auf. In einem Arbeitsstatus führt eine Steuerung beispielsweise die folgenden Aufgaben aus: Abspeichern der aktuellen Pose, Berechnen einer Pose, Abrufen von vorgespeicherten Posen, Schreiben von OP-Protokollen, Aufnehmen/Verarbeiten von mittels des Anbaugeräts erfassten Daten, Aufspielen von Software auf Mikrocontroller in Gelenken, Anzeigen einer Progress-Bar beim Arbeiten bzw. Verarbeiten von Daten, und dergleichen. Hierdurch erhält ein Bediener unmittelbar Feedback darüber, in welchem Status die Haltevorrichtung ist, beispielsweise ob die aktuell eingenommene Pose abgespeichert wird oder dergleichen. Der Bediener kann auch erkennen, ob Daten, die mittels des Anbaugeräts aufgenommen oder verarbeitet werden, gespeichert und/oder verarbeitet werden.

Gemäß einer weiteren Ausführungsform ist der Status ein Kommunikationsstatus der Haltevorrichtung mit dem Anbaugerät. Das heißt, die Anzeigeeinheiten zeigen an, ob und vorzugsweise wie, welche Intensität, Datenmenge oder dergleichen, die Haltevorrichtung mit dem Anbaugerät kommuniziert. Werden beispielsweise Daten von dem Anbaugerät an die Haltevorrichtung, insbesondere eine dafür vorgesehene Schnittstelle am distalen Ende, übertragen, so wird dies mittels der Anzeigeeinrichtung angezeigt. Dies kann beispielsweise durch Beleuchten wenigstens einer Anzeigeeinrichtung in einer vordefinierten Farbe erfolgen.

Weiterhin ist bevorzugt, dass der Status eine Bewegung der Haltevorrichtung ist. Bevorzugt ist demnach vorgesehen, dass die Anzeigevorrichtung anzeigt, wenn ein Gelenk bewegt wird. Gemäß dieser Ausführungsform ist beispielsweise vorgesehen, dass zunächst mittels einer Bedieneinheit ein Gelenk freigegeben wird, die Anzeigeeinheit aber nicht, bzw. nicht nur, das Freigeben anzeigt, sondern das tatsächliche Bewegen des Gelenks. Dazu weist der Haltearm vorzugsweise Lagesensoren in wenigstens einem, vorzugsweise allen, Gelenken auf.

Wenn der Status eine Bewegung ist, umfasst dies auch in einer bevorzugten Ausführungsform, dass die Anzeigevorrichtungen anzeigen, wenn die Haltevorrichtung insgesamt, insbesondere ohne Veränderung der Pose, bewegt wird. Dies ist beispielsweise dann der Fall, wenn die Haltevorrichtung mittels des proximalen Endes an einem OP-Tisch befestigt ist, und dieser bewegt, das heißt verschoben, verschwenkt, gedreht oder dergleichen, wird. In einem solchen Fall wird die Bewegung des Haltearms bevorzugt durch wenigstens eine Anzeigeeinheit angezeigt, beispielsweise durch Aufleuchten, Blinken, Ändern der Farbe, ein akustisches Signal oder dergleichen. Eine Veränderung der Position des OPTisches kann bedeuten, dass auch auf diesem angeordnete Gegenstände, oder auch der Patient, die Lage ändern, wodurch sich eine relative Lage der Haltevorrichtung zum Patienten ändern kann, wodurch wiederum Gefahren hervorgerufen werden können. Daher wird auch in dieser Ausführungsform die Sicherheit wesentlich verbessert.

Weiterhin umfasst der Status der Bewegung bevorzugt, eine Bewegung eines Gelenks im arretierten Zustand. Wird die Haltevorrichtung stark belastet, kann es vorkommen, dass einzelne Bremsen in Gelenken "durchrutschen", und sich ein Gelenk bewegt, obwohl es arretiert ist. Gemäß dieser Ausführungsform ist vorgesehen, dass eine derartige Bewegung durch insbesondere einen Lagesensor erfasst wird und die entsprechenden Anzeigeeinheiten diese Bewegung anzeigen. Dies erfolgt wiederum bevorzugt durch Aufleuchten, Ändern der Intensität des Aufleuchtens, Ändern der Farbe, Blinken, Ändern der Frequenz des Blinkens, ein Signalton oder dergleichen.

In einer weiteren bevorzugten Ausführungsform weist die Haltevorrichtung eine Messeinrichtung, zum Messen wenigstens eines physikalischen und/oder chemischen Werts auf, und der Status ist der gemessene Wert. Beispielsweise weist die Haltevorrichtung einen Kraftsensor auf, und an dem Anbaugerät, und/oder am distalen Ende der Haltevorrichtung greift eine Kraft an, beispielsweise aufgrund eines Kontakts zwischen Haltevorrichtung und einem Patienten, und der Status ist die gemessene Kraft. Ebenso könnte es beispielsweise der pH-Wert sein. Bevorzugt zeigt die Anzeigevorrichtung an, ob der gemessene Wert innerhalb vorbestimmter Grenzwerte liegt. Hierzu kann beispielsweise ein Zwei-Farben-Code, beispielsweise Rot-Grün verwendet werden. Zeigt die Anzeigeeinheit Rot an, ist dies gemäß einer Ausführungsform ein Indikator dafür, dass ein gemessener Wert, beispielsweise eine gemessene Kraft, außerhalb der vorbestimmten Grenzen liegt. Der Operateur sollte in einem solchen Fall die Pose der Haltevorrichtung anpassen, um so eine Pose zu erreichen, in der die Kraft in dem vorbestimmten Bereich liegt.

An dem distalen Ende der Haltevorrichtung, insbesondere an der Schnittstelle, sind bevorzugt mehrere Kraftsensoren angeordnet, um so eine Messeinrichtung zum Messen wenigstens eines physikalischen Werts zu bilden. Die mehreren Kraftsensoren sind bevorzugt so angeordnet, dass auch eine Momentenaufnahme an dem distalen Ende der Haltevorrichtung, insbesondere an einer Schnittstelle, gemessen werden kann. Alternativ kann auch ein einzelner Kraft-Momenten-Sensor dort vorgesehen sein. Dies ist besonders dann vorteilhaft, wenn am distalen Ende beispielsweise ein Retraktor oder ein ähnliches Werkzeug aufgenommen ist, welches mit einer vorbestimmten Kraft oder einer Kraft in einem vorbestimmten Bereich auf den Patienten oder ein anderes Objekt einwirken soll. Ein Bediener kann durch Bewegen der Haltevorrichtung die Kraft exakt einstellen, diese wird mittels der Anzeigeeinheiten angezeigt, und der Bediener kann die Haltevorrichtung so bewegen, dass die Gelenke dann arretiert werden, wenn die Kraft in etwa den vorbestimmten Wert erreicht hat. So ist es beispielsweise möglich, mit einer vorbestimmten Kraft an einem Gewebeteil zu ziehen. Gleichzeitig weist die Haltevorrichtung vorzugsweise eine Zeitmesseinheit auf, die misst, für welche Zeitdauer dieselbe oder eine ähnliche Kraft anliegt. Beispielsweise kann es vorteilhaft sein, an bestimmten Gewebeabschnitten des Patienten nur für einen bestimmten Zeitabschnitt mit einer bestimmten Kraft einzuwirken, und anschließend die Höhe der Kraft sowie die Richtung der Kraft zu ändern. Auch dies kann einem Bediener mittels der Anzeigeeinheiten angezeigt werden. So ist es beispielsweise vorteilhaft, wenn neben der Kraft auch die Zeitdauer angezeigt wird. Dadurch können Verletzungen des Patienten vermieden werden. Ferner werden Assistenzpersonen bei einer Operation entlastet und müssen keine lang andauernde statische Haltekraft (Kraft mit bestimmter Höhe in bestimmter Richtung) leisten. Zusätzlich kann vorgesehen sein, dass, wenn ein Operateur die Haltevorrichtung so bewegt, dass sich die Kraft erhöht, ein oder mehrere Gelenke von einer internen Steuereinheit geschlossen werden, wenn eine Maximalkraft erreicht ist. So wird verhindert, dass mit einer zu großen Kraft auf den Patienten eingewirkt wird. Gleichzeitig ist bevorzugt, dass das automatische Arretieren der Gelenke mittels der Anzeigeeinheiten angezeigt wird, sodass der Operateur hierüber eine Rückmeldung erhält. Dies hilft, die Traumatisierung zu verhindern.

Gemäß einer weiteren Ausführungsform sind die Anzeigevorrichtungen dazu eingerichtet, anzuzeigen, ob ein entsprechendes Gelenk freigegeben oder arretiert ist. Die Haltevorrichtung weist eine Bedieneinrichtung zum Freigegen und/oder Arretieren des entsprechenden Gelenks auf. Gemäß dieser Ausführungsform zeigen die Anzeigevorrichtungen zusätzlich an, ob ein entsprechendes Gelenk mittels der Bedieneinrichtung freigegeben oder arretiert ist.

Erfindungsgemäß wird die eingangs genannte Aufgabe einer Haltevorrichtung der eingangs genannten Art gelöst durch eine Bedieneinrichtung, die dazu eingerichtet ist, bei Kontakt zwischen einer Bedienperson und einem der ersten und zweiten Armsegmente das zugeordnete Gelenk freizugeben. Gemäß einer weiteren Ausführungsform sind die Anzeigeeinheiten dazu eingerichtet, anzuzeigen, ob ein entsprechendes Gelenk freigegeben oder arretiert ist. Die Haltevorrichtung gemäß diesem Aspekt weist erfindungsgemäß eine Bedieneinrichtung auf, die dazu eingerichtet ist, bei Kontakt zwischen einer Bedienperson und einem der ersten und zweiten Armsegmente das zugeordnete Gelenk freizugeben.

Gemäß dieser Ausführungsform ist daher vorgesehen, dass die Bedieneinrichtung dazu eingerichtet ist, bei Kontakt zwischen einer Bedienperson und dem ersten Armsegment, das erste Gelenk freizugeben und bei Kontakt zwischen einer Bedienperson und dem zweiten Armsegment, das zweite Gelenk freizugeben. Es ist also gemäß dieser Ausführungsform vorgesehen, dass bei Kontakt einer Bedienperson mit einem entsprechenden Armsegment nur das zugeordnete Gelenk freigegeben wird. Dieses Freigeben wird dann mittels den Anzeigeeinheiten angezeigt. Dadurch ist es möglich, intuitiv einzelne Gelenke zu bewegen und die Haltevorrichtung so segmentweise zu verstellen und in eine gewünschte Pose zu bringen. Dadurch ist eine genauere Positionierung möglich, da inkrementell jedes Segment separat verstellt werden kann. Es ist ebenso möglich, mehrere Segmente auch im Wesentlichen gleichzeitig zu kontaktieren, sodass mehrere Gelenke gleichzeitig freigegeben werden und dieses Freigeben gleichzeitig angezeigt wird, und die Gelenke verstellbar sind. Dadurch ist es möglich, die Haltevorrichtung auf einfache Art und Weise, insbesondere intuitiv, in eine gewünschte Pose zu überführen, wobei die Anzeigeeinheiten das Freigeben einzelner Gelenke anzeigen und der Bediener so ein unmittelbares und intuitives Feedback erhält.

Neben den ersten und zweiten Armsegmenten sind vorzugsweise weitere Armsegmente vorgesehen, die ebenfalls jeweils einem Gelenk zugeordnet sind. Die Armsegmente selbst sind im Wesentlichen starr und vorzugsweise im Wesentlichen stabförmig. Der Begriff "stabförmig" umfasst hier sowohl im Wesentlichen gerade Armsegmente als auch leicht bis stark gekrümmte Armsegmente. Bei einer derartigen Haltevorrichtung wechseln sich Armsegmente und Gelenke stets ab, wobei die Haltevorrichtung am distalen und am proximalen Ende sowohl mit einem Gelenk als auch mit einem Segment oder einem Anschlusselement enden kann. Mit dem proximalen Ende ist die Haltevorrichtung an einer Basis befestigbar. Die Basis kann alternativ fest mit der Haltevorrichtung gekoppelt sein, oder die Haltevorrichtung ist von der Basis entnehmbar. Die Basis ist in einer Ausführungsform als Operationstisch ausgebildet, und die Haltevorrichtung ist mit einem Operationstisch koppelbar. Vorzugsweise ist die Haltevorrichtung mit einer an dem Operationstisch vorgesehenen Normschiene koppelbar. Derartige Normschienen sind in der Regel bei Operationstischen vorgesehen, sodass an der Haltevorrichtung zur Kopplung mit der Normschiene eines Operationstisches eine Standardschnittstelle vorgesehen sein kann. Übliche Operationstische sind zudem aus einzelnen Segmenten zusammengesetzt. Zur Kopplung weisen die Segmente an den Stirnseiten entsprechende Koppelstellen auf, die in der Regel herstellerspezifisch sind. Bevorzugt ist die Haltevorrichtung über eine solche Koppelstelle an dem Operationstisch befestigbar. Dazu kann an dem proximalen Ende ein herstellerspezifischer Adapter vorgesehen sein. Alternativ ist die Basis als eine separate Vorrichtung, wie etwa ein Ständer, der auf dem Boden eines Operationssaales aufstellbar ist. In einer weiteren Alternative ist die Basis als eine Halterung ausgebildet, die beispielsweise an einer Wand oder Decke eines Operationssaales befestigbar ist.

Die Haltevorrichtung ist vorzugsweise als sogenannte passive Haltevorrichtung ausgebildet und weist daher ausschließlich aktiv gebremste Gelenke auf, jedoch keine angetriebenen Gelenke, wie dies bei robotischen Haltevorrichtungen häufig der Fall ist. Jedes Gelenk ist daher nur freigeb- und arretierbar, allerdings nicht antreibbar. Hierdurch ist die Haltevorrichtung einfach aufgebaut und bedarf keiner komplexen Steuerung zu ihrem Betrieb.

Gemäß einer ersten bevorzugten Ausführungsform der Erfindung weist die Bedieneinrichtung Kontaktmittel auf, die dazu vorgesehen sind, dass ein Bediener mit diesen in Kontakt kommt, wobei ein erstes Kontaktmittel der Bedieneinrichtung an dem ersten Armsegment angeordnet ist und ein zweites Kontaktmittel an dem zweiten Armsegment angeordnet ist. Bei Kontakt mit dem ersten Kontaktmittel wird vorzugsweise das erste Gelenk freigegeben, und bei Kontakt mit dem zweiten Kontaktmittel wird vorzugsweise das zweite Gelenk freigegeben. Die Kontaktmittel dienen dazu, den Kontakt zwischen dem Benutzer und dem Armsegment zu erfassen. Die Kontaktmittel sind vorzugsweise jeweils an einer Oberfläche des entsprechenden Armsegments angeordnet. Die Kontaktmittel können sich über das gesamte Armsegment erstrecken oder nur einen Abschnitt von diesem einnehmen. Vorzugsweise erstreckt sich das Kontaktmittel jeweils in etwa um einen halben Umfang bezogen auf eine Zentralachse eines Armsegments. Dadurch ist das Kontaktmittel in jeder Pose der Haltevorrichtung leicht zugänglich, und ein Bediener kann leicht mit diesem in Kontakt kommen.

Gemäß einer weiteren bevorzugten Ausführungsform weist jeder Kontaktabschnitt zwei, drei oder mehr an dem Armsegment im Wesentlichen gegenüberliegend bzw. gleichmäßig verteilt angeordnete Kontaktelemente auf. Gemäß diesem Ausführungsbeispiel ist es bevorzugt, dass das zugeordnete Gelenk nur bei Kontakt von den beiden bzw. zwei von drei oder mehr, oder allen Kontaktelementen freigegeben wird. Der Kontaktabschnitt besteht vorzugsweise aus den zwei, drei oder mehr Kontaktelementen, sodass ein Kontakt mit dem Kontaktabschnitt nur dann vorliegt, wenn wenigstens zwei Kontaktelemente von dem Bediener kontaktiert werden. Indem die beiden Kontaktelemente im Wesentlichen gegenüberliegend, vorzugsweise bezogen auf eine Ebene, die eine Zentralachse des Armsegments enthält, angeordnet sind, ist es möglich, einen unbeabsichtigten Kontakt, beispielsweise durch den Arm eines Bedieners von einem beabsichtigten Kontakt, einem konkreten Greifen des Armsegments zu unterscheiden; folglich wird gemäß diesem Ausführungsbeispiel das Gelenk nur bei einem Ergreifen des Armsegments, insbesondere durch die Hand des Bedieners, bei dem die beiden gegenüberliegenden Seiten des Armsegments kontaktiert werden, freigegeben. Zur Bedienung des Haltearms und zum Verbringen der Haltevorrichtung in eine gewünschte Pose mittels der Bedieneinrichtung ist folglich das Armsegment durch den Bediener so zu ergreifen, dass er in Kontakt mit den beiden bzw. drei Kontaktelementen des Kontaktabschnitts kommt, woraufhin dann durch die Bedieneinrichtung das zugeordnete Gelenk freigegeben wird, und das Armsegment bewegt werden kann.

Gemäß einer bevorzugten Weiterbildung sind die Kontaktelemente als Taster ausgebildet. Taster sind besonders einfache Elemente, die sowohl optisch durch den Bediener erfasst werden können, als auch unmittelbar ein taktiles Feedback beim Drücken des Tasters bieten. Ein solcher Taster kann beispielsweise als einfacher Schließer eines elektrischen Kreislaufs oder als kapazitiver Schalter ausgebildet sein. Solange gemäß diesem Ausführungsbeispiel beide Taster gedrückt sind, wird das dem entsprechenden Armsegment zugeordnete Gelenk freigegeben; sobald der Bediener die beiden oder auch nur einen der beiden Taster loslässt, wird das Gelenk durch die Bedieneinrichtung wieder arretiert.

Gemäß einer bevorzugten alternativen Ausführungsform sind die Kontaktmittelelemente als berührungsempfindliche Sensoren ausgebildet. Die Sensoren sind vorzugsweise im Wesentlichen flächig ausgebildet und erstrecken sich über einen wesentlichen Abschnitt der Oberfläche des entsprechenden Armsegments. Vorzugsweise sind die Sensoren als druckempfindliche Sensoren, kapazitive Sensoren, wärmeempfindliche Sensoren und/oder optische Sensoren ausgebildet. Derartige Sensoren haben den Vorteil, dass sie eine größere Fläche abdecken können, der Bediener also das Armsegment nicht so genau kontaktieren muss, sondern es ausreicht, dass der Bediener das Armsegment im Wesentlichen umgreift und so mit dem Sensor bzw. den Sensoren in Kontakt kommt.

In einer weiteren bevorzugten Ausgestaltung ist die Bedieneinrichtung dazu ausgebildet, in Abhängigkeit der Intensität des Kontakts das zugeordnete Gelenk freizugeben. Intensität bezieht sich hier auf einen Druck und/oder eine Kraft, der bzw. die durch den Bediener aufgebracht wird. So ist es möglich, dass der Bediener durch die Kraft, die er beim Zugreifen aufbringt, einen Grad der Freigabe steuert. So ist es denkbar und bevorzugt, dass das zugeordnete Gelenk bei einer geringen Intensität des Kontakts nur teilweise freigegeben wird, sodass das Armsegment nur langsam und gegen einen Widerstand bewegbar ist. Bei einer hohen Intensität und demzufolge einem festen Zugreifen wird das Gelenk dann vollständig geöffnet, sodass das Armsegment im Wesentlichen widerstandsfrei beweglich ist. Ein teilweises Freigeben kann auch durch ein intermittierendes Freigeben in verschiedenen Frequenzen realisiert werden.

Es ist vorzugsweise vorgesehen, dass bei Kontakt zwischen einer Bedienperson und einem oder mehreren Armsegmenten ein, mehrere oder sämtliche Gelenke, insbesondere die Gelenke, die zwischen zwei kontaktierten Armsegmenten liegen, freigegeben werden.

In einem weiteren Aspekt wird die eingangs genannte Aufgabe einem Verfahren der eingangs genannten Art zum Anzeigen wenigstens eines von einem Freigeben und Arretieren eines Gelenks einer Haltevorrichtung, insbesondere einer Haltevorrichtung nach einer der vorstehend beschriebenen bevorzugten Ausführungsformen, verschiedenen Statussen der Haltevorrichtung und/oder eines Anbaugeräts gelöst, mit den Schritten: Erfassen des Statusses; Anzeigen des Statusses, insbesondere mittels einer im Wesentlichen ringförmig um eine Schwenkachse eines entsprechenden Gelenks ausgebildeten Anzeigeeinheit.

Es soll verstanden werden, dass der dritte Aspekt der Erfindung und der erste und/oder zweite Aspekt der Erfindung gleiche oder ähnliche Aspekte umfassen, wie sie insbesondere in Unteransprüchen dargelegt sind. Insofern wird vollumfänglich auf die obige Beschreibung verwiesen.

Nachstehend wird die Erfindung anhand eines Ausführungsbeispiels unter Bezugnahme auf die beiliegenden Figuren näher erläutert. Dabei zeigen:
- Figur 1: eine Seitenansicht einer Haltevorrichtung gemäß der Erfindung;
- Figur 2: eine perspektivische Ansicht der Haltevorrichtung aus Figur 1 mit am distalen Ende aufgenommenen Anbaugerät;
- Figuren 3a, 3b: zwei schematische Ansichten einer Anzeigeeinheit;
- Figuren 4a bis 4c: drei schematische Ansichten einer Anzeigeeinheit;
- Figuren 5a bis 5c: drei schematische Ansichten einer Anzeigeeinheit;
- Figuren 6a bis 6c: drei schematische Ansichten einer Anzeigeeinheit;
- Figuren 7a bis 7c: drei schematische Ansichten einer Anzeigeeinheit;
- Figuren 8a, 8b: eine weitere Ausführungsform einer Anzeigeeinheit;
- Figuren 9a, 9b: eine weitere Ausführungsform einer Anzeigeeinheit;
- Figur 10: ein weiteres Ausführungsbeispiel einer Haltevorrichtung;
- Figur 11: eine weitere Ansicht der Haltevorrichtung aus Figur 10;
- Figur 12: eine weitere Ansicht der Haltevorrichtung aus Figur 10;
- Figur 13: ein schematisches Diagramm für ein Verfahren, das den Aufbau der Haltevorrichtung gemäß der Erfindung zeigt; und
- Figur 14: ein schematisches Diagramm für ein Verfahren, das den Aufbau der Haltevorrichtung gemäß der Erfindung zeigt.

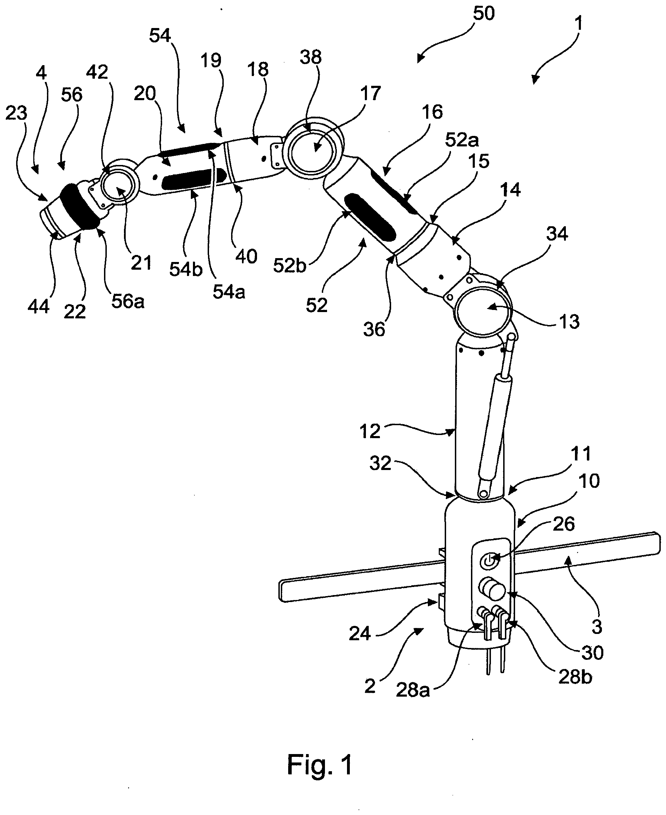

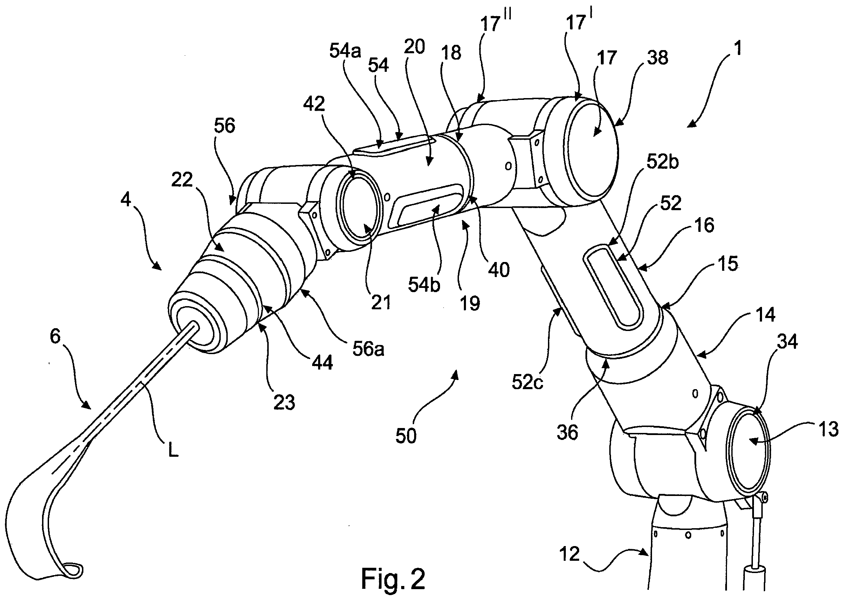

Gemäß Figur 1 ist eine Haltevorrichtung 1, in Form eines Haltearms gezeigt. Die Haltevorrichtung weist ein proximales Ende 2 zur Befestigung der Haltevorrichtung 1 an einer Basis 3 auf. Die Basis 3 ist gemäß diesem Ausführungsbeispiel als Normschiene eines Operationstisches ausgebildet (der Operationstisch ist in Figur 1 nicht gezeigt). Die Haltevorrichtung 1 weist ferner ein distales Ende 4 zum Aufnehmen eines Anbaugeräts 6 (vergleiche Figur 2) auf.

Die Haltevorrichtung gemäß den Figuren 1 und 2 weist sieben Armsegmente 10, 12, 14, 16, 18, 20, 22 auf, wobei zwischen den einzelnen Armsegmenten 10 bis 22 die Gelenke 11, 13, 15, 17, 19, 21, 23 vorgesehen sind. Das erste Armsegment 10 bildet das proximale Ende 2 und weist eine Klimmklaue 24 auf, mittels der die Haltevorrichtung 1 an der Basis 3 festlegbar ist. An dem Armsegment 10 ist ferner ein Einschaltknopf 26 vorgesehen zum Einschalten der gesamten Haltevorrichtung, zwei Anschlüsse 28a, 28b über die die Haltevorrichtung mit Strom und Daten, wie beispielsweise Stellsignale und dergleichen, versorgbar ist, und über die die Daten von der Haltevorrichtung an externe Einheiten, wie an OP-Systeme übertragen werden, sowie ein Notausschalter 30.

Die Gelenke 11, 15, 19 und 23 sind als rotatorische Gelenke ausgebildet und die Gelenke 13, 17 und 21 als Schwenkgelenke. Das heißt, bezogen auf Figur 1, liegen die Drehachsen der Gelenke 11, 15, 19 und 23 im Wesentlichen innerhalb der Zeichenebene, während sich die Drehachsen der Gelenke 13, 17 und 21 im Wesentlichen senkrecht zur Zeichenebene erstrecken.

Die Haltevorrichtung 1 weist an jedem Gelenk 11, 13, 15, 17, 19, 21, 23 eine Anzeigeeinheit 32, 34, 36, 38, 40, 42, 44 auf, die dazu vorgesehen sind, einen Status der Haltevorrichtung und/oder eines Anbaugeräts (vergleiche Figur 2) anzuzeigen.

Die Anzeigeeinheiten 32, 34, 36, 38, 40, 42, 44 sind gemäß diesem Ausführungsbeispiel als im Wesentlichen ringförmige Lichtquellen, insbesondere als LED-Ringe ausgebildet. Die Zentralachse von jedem Ring verläuft im Wesentlichen koaxial zur jeweiligen Drehachse des Gelenks 11, 13, 15, 17, 19, 21, 23. Während für die Gelenke 11, 15, 19, 23 jeweils ein einziger LED-Ring vorgesehen ist, sind für die Gelenke 13, 17 und 21 jeweils zwei LED-Ringe vorgesehen. Die zwei LED-Ringe sind an vorderen und hinteren Gelenkabschnitten 17', 17" (in Figur 2 nur beispielsweise mit Bezugszeichen versehen) vorgesehen. So ist in jeder Lage der Haltevorrichtung jede Anzeigeeinheit stets erkennbar.

Erfindungsgemäß (vergleiche Figuren 1 und 2) weist die Haltevorrichtung ferner eine Bedieneinrichtung 50 auf. Mittels der Bedieneinrichtung 50 ist der Haltearm in eine gewünschte Pose verbringbar, wobei die Bedieneinrichtung 50 dazu eingerichtet ist, bei Kontakt zwischen einer Bedienperson und einem der sieben Armsegmente 10, 12, 14, 16, 18, 20, 22, das zugeordnete Gelenk 11, 13, 15, 17, 19, 21, 23 freizugeben. Dazu weist die Bedieneinrichtung 28 gemäß diesem Ausführungsbeispiel drei Kontaktabschnitte 52, 54, 56 auf, wobei jeder Kontaktabschnitt 52, 54, 56 an einem anderen Armsegment 16, 20, 22 angeordnet ist. So ist am Armsegment 16 ein Kontaktabschnitt 52, am Armsegment 20 ein Kontaktabschnitt 54 und am Armsegment 22 ein Kontaktanschnitt 56 angeordnet. Jeder Kontaktabschnitt 52, 54, 56 weist separate Kontaktelemente 52a, 52b, 52c, 54a, 54b, 54c und 56a auf. Die einzelnen Kontaktelemente sind als berührungsempfindliche Oberflächen ausgebildet, sodass bei Kontakt zwischen einer Bedienperson und einem entsprechenden Kontaktmittel ein oder mehrere zugeordnete Gelenke freigegeben werden.

Gemäß diesem Ausführungsbeispiel sind an den Armsegmenten 16 und 20 jeweils drei Kontaktelemente 52a, 52b, 52c, 54a, 54b, 54c ausgebildet, an dem Armsegment 22 ist ein ringförmiges Kontaktelement 56 angeordnet, welches auch um seine Zentralachse drehbar ist, um so Funktionen an eine Schnittstelle am distalen Ende 4 aufgenommenen Anbaugeräts zu beeinflussen.

Die Zuordnung der einzelnen Gelenke 11, 13, 15, 17, 19, 21, 23 ist gemäß diesem Ausführungsbeispiel wie folgt geregelt: Bei Kontakt zwischen einer Bedienperson und dem Armsegment 16, also den Kontaktelementen 52a, 52b, 52c des Kontaktabschnitts 52 werden die Gelenke 15, 13 und 11 freigegeben. Eine Bedienperson kann nun drei Freiheitsgrade beeinflussen; dies ist ein Umfang, der manuell gut beherrschbar ist und in den manuell die Haltevorrichtung in eine gewünschte Pose bringbar ist. Kommt eine Bedienperson mit dem Armsegment 16 in Kontakt, und werden die Gelenke 15, 13 und 11 freigegeben, ist bevorzugt vorgesehen, dass die entsprechenden Anzeigeeinheiten 32, 34, 36 dieses Freigeben anzeigen, gemäß dem Ausführungsbeispiel der Figuren 1 und 2, also durch Aufleuchten des LED-Rings.

Bei Kontakt zwischen dem Armsegment 20, also dem Kontaktabschnitt 54 und insbesondere dem Kontaktmitteln 54a, 54b, 54c, werden die Gelenke 19 und 17 freigegeben. Entsprechend ist bevorzugt vorgesehen, dass die Anzeigeeinheiten 36, 38 dies anzeigen. Schließlich wird bei Kontakt zwischen dem Armsegment 22, also dem Kontaktabschnitt 56 und insbesondere dem Kontaktelement 56a die Gelenke 21 und 23 freigegeben, was bevorzugt mittels den Anzeigeeinheiten 42, 44 angezeigt wird.

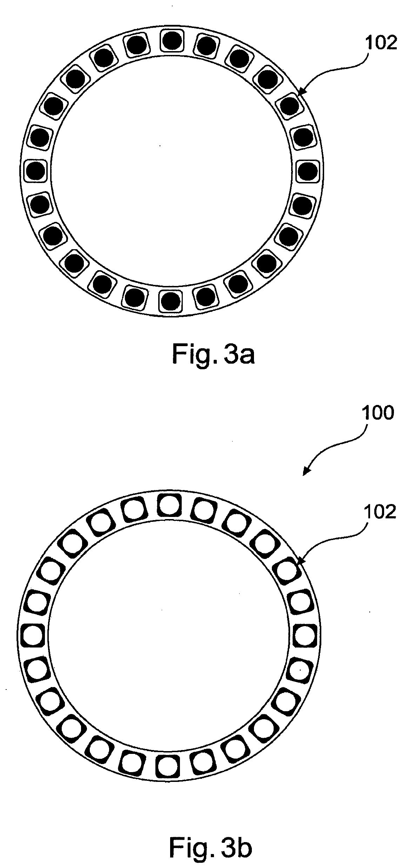

Nun bezugnehmend auf Figur 2 ist ein Anbaugerät 6 in Form eines Retraktors bzw. Wundspreizer an dem distalen Ende 4 aufgenommen. An der Schnittstelle am distalen Ende 4, an der der Retraktor 6 aufgenommen ist, sind eine oder mehrere Kraftsensoren angeordnet, mittels denen eine in Richtung der Längsachse L wirkende Zugkraft bestimmt werden kann. Ferner können mittels dieser Sensoren bevorzugt auch entsprechende Momente an der Schnittstelle um die Längsachse L, als auch senkrecht zu dieser, bestimmt werden. Die Anzeigeeinheit 44 ist dazu eingerichtet, diesen Status des Anbaugeräts 6 anzuzeigen, und insbesondere anzuzeigen, ob eine bestimmte Kraft innerhalb vorbestimmter Grenzen liegt. Bei Operationen besteht die Gefahr, dass ein Retraktor 6 über eine längere Zeit mit einer zu hohen Kraft beaufschlagt wird, wodurch das Gewebe, welches von dem Operationsfeld weggehalten wird, negativ beeinflusst wird. Durch Messen dieser Kraft und Bestimmung, ob diese innerhalb vorbestimmter Grenzen liegt, kann dieses Problem verringert bzw. vermieden werden. Die Figuren 3a bis 7c zeigen insgesamt verschiedene Ausführungsbeispiele von Anzeigeeinheiten gemäß der Erfindung. Alle in den Figuren 3a bis 7c dargestellten Anzeigeeinheiten sind einreihig ausgebildet. Figur 3a zeigt eine Anzeigeeinheit 100 in einem bevorzugten Ausführungsbeispiel der Erfindung. Die Anzeigeeinheit 100 ist ringförmig und weist eine Mehrzahl an LEDs 102 (in Figur 3a nur eine mit Bezugszeichen versehen) auf, die ringförmig angeordnet sind. Die LEDs 102 sind gemäß dem Ausführungsbeispiel aus Figur 3a, 3b innerhalb der Zeichenebene ausgerichtet, sodass die Anzeigeeinheit gemäß diesem Ausführungsbeispiel etwa als Anzeigeeinheit 34, 38 oder 42 eingesetzt werden kann. Während die Anzeigeeinheit 100 in Figur 3a in einem ersten Zustand gezeigt wird, ist dieselbe Anzeigeeinheit 100 in Figur 3b in einem zweiten Zustand gezeigt. Es soll verstanden werden, dass die beiden Zustände beispielsweise einen Aus-Zustand in Figur 3a und einen An-Zustand in Figur 3b sein können. Alternativ ist auch denkbar, dass Figur 3a zeigt, dass die Anzeigeeinheit 100 in einer ersten Farbe leuchtet, während sie in Figur 3b in einer zweiten abweichenden Farbe leuchtet. Eine derartige Visualisierung wird insbesondere zum Anzeigen des freigegebenen und/oder arretierten Zustands der Haltevorrichtung verwendet, also um anzuzeigen, ob ein entsprechendes Gelenk gesperrt oder freigegeben ist. Hierzu sind verschiedene Ausführungsvarianten denkbar und bevorzugt. In einer ersten Ausführungsform ist die Anzeigeeinheit 100 gemäß Figuren 3a, 3b mit einer internen Steuerung der Haltevorrichtung 1 (vgl. Figuren 1, 2) verbunden. Hierbei ist bevorzugt, dass die Anzeigevorrichtung 100 in einer ersten Farbe aufleuchtet, wenn eine Berührung zwischen der Bedienperson und einer der Kontaktelemente 52a, 52b, 52c, 54a, 54b, 54c, 56a erfasst wird. Hierbei ist vorzugsweise vorgesehen, dass bei Kontakt nicht unmittelbar das entsprechende Gelenk freigegeben wird, sondern dies zeitverzögert erfolgt, beispielsweise nach zwei Sekunden. Dadurch wird mittels der Anzeigeeinheit nicht das Freigeben bzw. Arretieren eines Gelenks angezeigt, sondern die Betätigung der Bedieneinrichtung, sodass ein Bediener noch ausreichend Zeit hat, seine Handlung abzubrechen oder zu bestätigen, bis das Gelenk tatsächlich freigegeben wird. Es kann auch vorgesehen sein, dass wenn alle Gelenke freigegeben werden, nur eine Anzeigeeinheit 100 aufleuchtet. In einer weiteren bevorzugten Ausführungsform ist in dem Armsegment 10 (siehe Figur 1) eine zusätzliche Anzeigeeinheit vorgesehen, die keinem Gelenk zugeordnet ist. Diese zeigt an, dass sämtliche Gelenke in der Haltevorrichtung freigegeben sind. In einem solchen Fall ist es denkbar, dass die einzelnen, den Gelenken zugeordneten Anzeigeeinheiten 32, 34, 36, 38, 40, 42, 44 nicht separat das Freigeben der Gelenke anzeigen.

In einer weiteren bevorzugten Ausführung des Ausführungsbeispiels (Figuren 3a, 3b) sind die Anzeigeeinheiten 100 mit einer Signalleitung der Bremsen in den Gelenken gekoppelt. Ein Aufleuchten einer Anzeigeeinheit 100 in einem solchen Ausführungsbeispiel erfolgt dann, wenn eine Spannung an die Bremse angelegt wird, um diese zu öffnen. Alternativ oder zusätzlich ist die Anzeigeeinheit 100 mit einem Bussystem der Bremsen gekoppelt, sodass die Anzeigeeinheiten das Stellsignal für die Bremse abgreifen und aufgrund dieses Stellsignals aufleuchten, und so anzeigen, dass die Bremse ein Stellsignal empfangen hat, um freigegeben zu werden.

Das in den Figuren 3a, 3b gezeigte Ausführungsbeispiel, in dem eine Anzeigeeinheit 100 zwischen zwei oder mehr verschiedenen Farben schaltet (insbesondere vollständig schaltet, das heißt alle LEDs 102 haben die selbe Farbe), wird bevorzugt auch dazu eingesetzt, um einen vom Freigeben und Arretieren des entsprechenden Gelenks verschiedenen Status der Haltevorrichtung oder des Anbaugeräts anzuzeigen. Sowohl ist bevorzugt, dass je nach Anwendung in der die Haltevorrichtung verwendet wird, die Anzeigeeinheiten 100 in einer dafür vorgesehenen Farbe leuchten. Wird die Haltevorrichtung beispielsweise in einer HNO-Operation verwendet, sind (vorzugsweise bei arretierten Bremsen) alle Anzeigeeinheiten grün. Wird dieselbe Haltevorrichtung in einer bauchchirurgischen Operation verwendet, sind alle Anzeigeeinheiten (vorzugsweise bei arretierten Bremsen) blau. Hierdurch ist ein Operateur in der Lage, unmittelbar zu erkennen, ob die Haltevorrichtung für die vorliegende Anwendung korrekt eingestellt ist, bzw. korrekt mit Daten von einem OP-System versorgt wird. Dies kann dann vorteilhaft sein, wenn die verschiedenen Anwendungen, beispielsweise unterschiedliches Bremsverhalten der Bremsen in den Gelenken erfordern, verschieden hohe Kräfte auf die Haltevorrichtung wirken können, oder nur eine bestimmte Anzahl und Gruppe an Anbaugeräten zulässig ist. Über Schnittstellen, vorzugsweise einem distalen und einem proximalen Ende der Haltevorrichtung 1 werden hierzu entsprechende Daten übergeben und/oder abgefragt und in einer internen Steuerung verarbeitet, die dann ein entsprechendes Signal an die Anzeigeeinheiten 100 sendet.

In einer weiteren Variante ist die Anzeigeeinheit 100 gemäß Figuren 3a, 3b mit einem oder mehreren Positionssensoren in den Gelenken, vorzugsweise die jeweilige Anzeigeeinheit mit einem Positionssensor in dem jeweiligen Gelenk gekoppelt. Der Status, der vom Arretieren und/oder Freigeben verschieden ist, ist in einer solchen Ausführungsform der Status der Bewegung eines Gelenks. Das heißt, ein Bediener kann zunächst mittels der Bedieneinheit 50 ein oder mehrere Gelenke freigeben, dies wird dann nicht durch die Anzeigeeinheit (N) angezeigt. Erst wenn der Bediener ein Gelenk bewegt, leuchtet die entsprechende Anzeigeeinheit auf. So ist es denkbar, dass der Bediener das Armsegment 20 greift (vgl. Figur 1) und dabei in Kontakt kommt mit dem Kontaktabschnitt 54. Dabei werden sowohl Gelenk 19 als auch 17 und 15 freigegeben. Verschwenkt der Bediener dann nur Gelenk 17, leuchtet nur die Anzeigeeinheit 38 auf. Dadurch erhält ein Bediener Feedback, welches Gelenk hier gerade bewegt wird und kann so seine Handlung überprüfen.

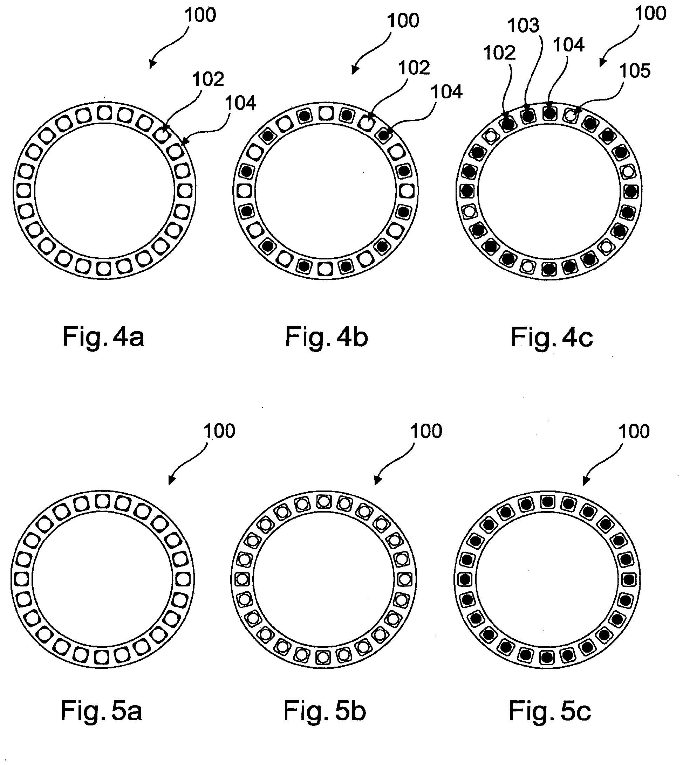

Die Figuren 4a bis 4c sowie 5a bis 5c zeigen eine Anzeigeeinheit 100, wie sie grundsätzlich aus den Figuren 3a, 3b bekannt ist, in einem zweiten Ausführungsbeispiel. In den Figuren 4a bis 5c sind Anzeigeeinheiten dargestellt, die nicht nur zwischen zwei oder mehr verschiedenen Farben insgesamt schalten können, sondern bei denen einzelne LEDs 102, 103, 104, 106 verschiedene Farben annehmen können (vgl. Figuren 4a bis 4c) bzw. die Intensität der Beleuchtung geändert wird (vgl. Figuren 5a bis 5c). Dies ist insbesondere dann vorteilhaft, wenn ein Positionieren der Haltevorrichtung erfolgt und der Bediener über dieses Positionieren ein Feedback erhalten soll. In einer Variante ist bevorzugt, dass eine Anzeigeeinheit 100, wie in den Figuren 5a bis 5c gezeigt, dann aufleuchtet, wenn ein Gelenk bewegt wird, das heißt, wenn beispielsweise Gelenk 17 bewegt wird, leuchtet die Anzeigeeinheit 38 auf. Je nach Geschwindigkeit der Bewegung kann dann die Leuchtintensität variiert werden. Figur 5a zeigt in einem solchen Fall eine Anzeigeeinheit 100, die anzeigt, dass keine Bewegung vorliegt, Figur 5b illustriert eine Bewegung mit mittlerer Geschwindigkeit und Figur 5c eine Bewegung mit hoher Geschwindigkeit.

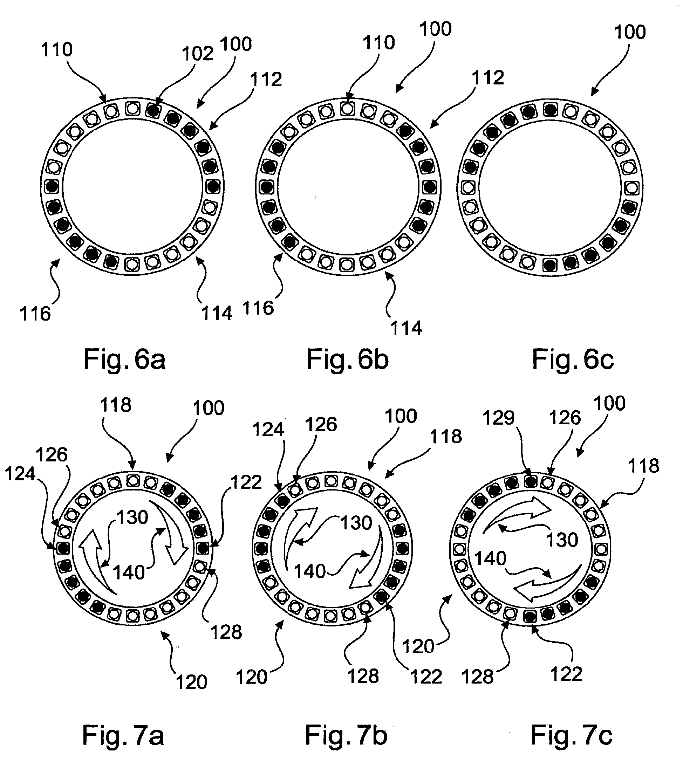

Positionssensoren in den Gelenken der Haltevorrichtung 1 werden bevorzugt auch zum Messen, insbesondere Vermessen einer Bewegung, verwendet. So ist es möglich, durch entsprechende Betätigung der Haltevorrichtung 1 und Bewegung der Haltevorrichtung 1 beispielsweise einen Abstand zwischen zwei Punkten, beispielsweise Gewebeabschnitten eines Patienten, zu bestimmen. Wird die Haltevorrichtung, die derart ausgebildet ist, in einen entsprechenden Modus zum Messen versetzt, ist es bevorzugt, dass die Anzeigeeinheiten dies anzeigen. Das ist beispielsweise in den Figuren 4a bis 4c dargestellt. Die Figuren 4a bis 4c illustrieren auch eine Funktion, bei der ein Bediener die Haltevorrichtung von einer aktuellen Pose in eine gewünschte Pose überführt und der angezeigte Status ist in einem solchen Fall der Abstand zur gewünschten Pose. Während in Figur 4a sämtliche LEDs 102, 104 in einer Farbe leuchten und so angezeigt wird, dass die aktuelle Pose nicht mit der gewünschten Pose übereinstimmt, zeigt Figur 4b, dass die Anzeigeeinheit 100 zeigt, dass der Bediener den Haltearm in der Richtung der gewünschten Pose bewegt hat; jede zweite LED 102, 104 hat eine verschiedene Farbe, das heißt, die Hälfte leuchtet in einer ersten Farbe und die andere Hälfte in einer zweiten Farbe. Figur 4c illustriert, dass der Bediener dann die Haltevorrichtung noch weiter Richtung gewünschter Pose bewegt hat; hierbei ist nur noch jede vierte LED 106 die erste Farbe, während die LEDs 102, 103, 104 bereits die zweite Farbe angenommen haben. Bei Erreichen der gewünschten Pose haben dann alle LEDs 102, 103, 104, 106 in die zweite Farbe gewechselt und der Bediener erkennt, dass er die gewünschte Pose erreicht hat. Vorzugsweise ist vorgesehen, dass bei Erreichen der gewünschten Pose alle Bremsen in den Gelenken automatisch geschlossen werden, also alle Gelenke arretiert werden. Die Figuren 6a bis 6c illustrieren eine weitere Möglichkeit der Anzeigeeinheit, eine Messfunktion bzw. einen Messmodus anzuzeigen. Die Anzeigeeinheit 100, in Form eines LED-Rings, hat vier verschiedene Abschnitte 110, 112, 114, 116, die zwei verschiedene Farben aufweisen, jeweils abwechselnd. Die einzelnen LEDs 102 (in den Figuren 6a bis 6c nur eine mit Bezugszeichen versehen) werden dann von einer internen Steuerung derart gesteuert, dass sich das Muster mit den vier Abschnitten 110, 112, 114, 116 mit Bezug auf die Figuren 6a bis 6c nach rechts dreht und so die einzelnen Abschnitte 110, 112, 114, 116 mit der Bewegung des entsprechenden Armsegments "wandern". Wird beispielsweise Gelenk 17 freigeschaltet und der Abschnitt der Haltevorrichtung zwischen dem Gelenk 17 und dem distalen Ende 4 verschwenkt, bewegen sich die einzelnen Abschnitte 110, 112, 114, 116 in Richtung der Schwenkbewegung, in der entsprechenden Geschwindigkeit, umso den Status der Bewegung anzuzeigen und den Bediener dabei zu unterstützen, zu erkennen, wie rasch und über welchen Winkel erden Haltevorrichtungsabschnitt verschwenkt.

Eine ähnliche Illustration ist in den Figuren 7a bis 7c gezeigt, in denen die Anzeigevorrichtung 100, die als LED-Ring ausgebildet ist, zwei Abschnitte 118, 120 aufweist. Diese weisen jeweils einen Farbverlauf auf von einer LED 122, 124, die in einer ersten Farbe leuchtet, bis hin zu einer LED 126, 128, die in einer zweiten Farbe leuchtet. Die Pfeile 130, 140 zeigen eine Rotationsrichtung des Musters an.

Weitere Ausführungsformen einer Visualisierung mittels einer Anzeigeeinheit 100 (wie insbesondere in den Figuren 3a, 3b gezeigt) sind, dass die Anzeigeeinheit 100 in einer ersten Farbe leuchtet, solange der Arm nicht schwingt, bzw. in einem definierten, abgespeicherten Toleranzfeld schwingt. Die erste Farbe bleibt solange angezeigt, bis der Arm nicht mehr schwingt. Dies ist dann bevorzugt, wenn Feinstmanipulatoren als Anbaugeräte an der Haltevorrichtung aufgenommen sind. Hierdurch wird ein Bediener informiert, abzuwarten, bis die Haltevorrichtung in einem Toleranzbereich ist, der akzeptabel ist. Wenn die Haltevorrichtung schwingt, dann soll möglichst ein Anbaugerät nicht weiter bewegt werden. Weiterhin ist mittels der Positionssensoren in den Gelenken erfassbar, wenn die Haltevorrichtung angestoßen wird, bzw. ein Gelenk im arretierten Zustand gegen die Kraft der Bremse verschwenkt wird. Dies ist mittels Positions- und/oder Lagesensoren erfassbar, und mittels den Anzeigeeinheiten anzeigbar. Hierdurch erhält ein Bediener Feedback, ob die Haltevorrichtung noch in der gewünschten Pose ist, oder ob etwa ein Gelenk gegen die Kraft der Bremse bewegt wurde.

Eine Anzeigeeinheit wie in den Figuren 6a bis 7c gezeigt, wird vorzugsweise auch dazu eingesetzt, ein Teachen, einer Abfolge von Posen (Trajektorie) an die Haltevorrichtung zu übergeben. Es kann vorteilhaft sein, im Vorfeld von einer Operation oder dergleichen bei der Haltevorrichtung verschiedene Posen abzufahren, und diese abzuspeichern und/oder zu testen. Hierzu ist es möglich und bevorzugt, die Haltevorrichtung in einen "Teach-Modus" zu versetzen, in dem die abgefahrenen Posen durch die interne Steuerung erfasst und gespeichert werden. Hierbei zeigen die Anzeigeeinheiten diesen Status an, insbesondere mit einem Muster, wie in den Figuren 7a bis 7c gezeigt.

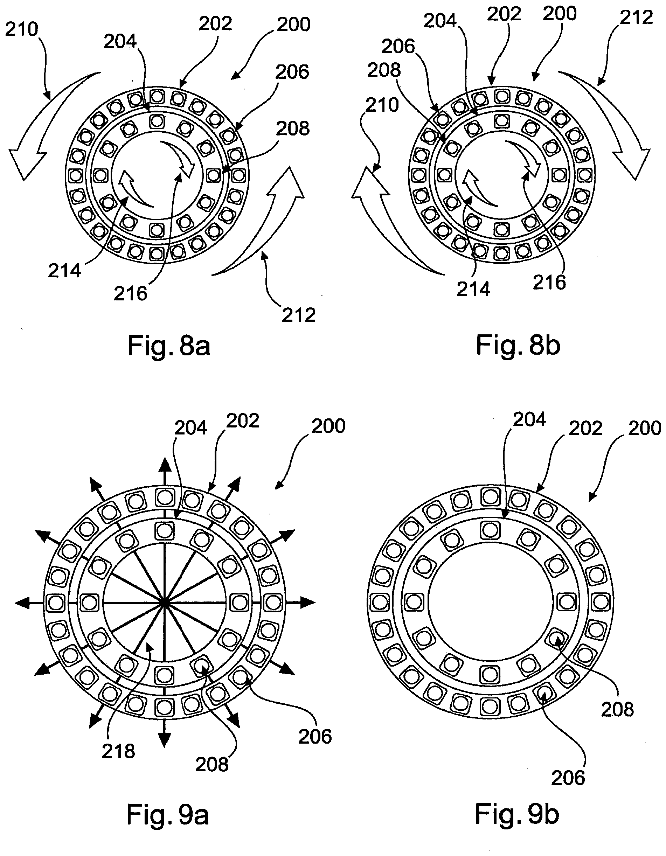

Die Figuren 8a bis 9b illustrieren eine Anzeigeeinheit 200 gemäß einem weiteren Ausführungsbeispiel, die als zwei-reihiger LED-Ring ausgebildet ist. Die Anzeigeeinheit 200 (vgl. Figur 8a) weist einen ersten LED-Ring 202 und einen zweiten LED-Ring 204 auf. In jedem LED-Ring 202, 204 sind eine Mehrzahl an LEDs 206, 208 (jeweils nur eine mit Bezugszeichen versehen) angeordnet. In einer solchen Ausführungsform ist es möglich, die beiden LED-Ringe 202, 204 unabhängig voneinander zu steuern. Es soll verstanden werden, dass nicht jeder LED-Ring 202, 204 ausschließlich an einer Reihe LEDs gebildet sein muss, sondern dass auch dieser wiederum mehrreihig sein kann, wobei die Reihen dann bevorzugt gleichgesteuert sind. Es soll auch verstanden werden, dass Ausführungsformen mit drei oder mehr LED-Ringen bevorzugt sind.

Gemäß den Figuren 8a, 8b ist beispielsweise gezeigt, dass ein rotierendes Muster auf dem LED-Ring 202, 204 angezeigt wird, beispielsweise wie dies mit Bezug auf die Figuren 6a bis 7c beschrieben wurde. Die Pfeile 210, 212 zeigen in den Figuren 8a, 8b eine Rotationsrichtung am äußeren LED-Ring 202 an, während die Pfeile 214, 216 die Drehrichtung des Musters am inneren LED-Ring 204 illustrieren. Während in Figur 8a dargestellt ist, dass das Muster gegenläufig bewegt wird (die Pfeile 210, 212 und 214, 216 sind gegenläufig angeordnet), illustriert Figur 8b, eine richtungsgleiche Rotation. Eine solche Anzeige (Figur 8a) ist beispielsweise bevorzugt, wenn zwei Gelenke freigegeben sind und diese gegensinnig bewegt werden, oder bewegt werden sollen, um eine Soll-Pose zu erreichen. Bei einer gleichläufigen Anzeige (Figur 8b) kann dies dazu verwendet werden, anzuzeigen, dass eine gleichsinnige Bewegung erforderlich ist.

Die Figuren 9a, 9b illustrieren eine Bewegung eines Musters radial von innen nach außen, wie durch die Pfeile 218 angezeigt. Ein solcher Effekt wird noch verstärkt, wenn drei oder mehr LED-Ringe vorgesehen sind. Auch eine Visualisierung mit einem Muster, welches von außen nach innen läuft, also entgegen der Pfeilrichtung der Pfeile 218, ist denkbar bevorzugt. Eine derartige Visualisierung wird insbesondere bevorzugt dazu eingesetzt, ein Softwareupdate von Softwarebausteinen von einem oder mehreren Gelenken, oder einer zentralen Steuereinheit anzuzeigen.

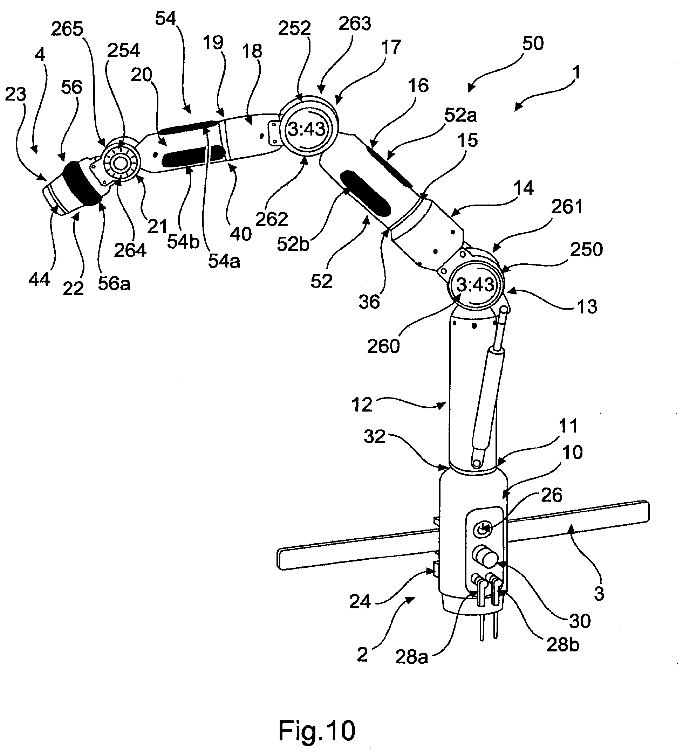

Figur 10 illustriert ein weiteres Ausführungsbeispiel der Haltevorrichtung 1. Diese stimmt in einer Vielzahl von Merkmalen mit der Haltevorrichtung 1 gemäß Figuren 1 und 2 überein, sodass gleiche und ähnliche Elemente mit den gleichen Bezugszeichen wie in den Figuren 1 und 2 bezeichnet werden. Insofern wird vollumfänglich auf die obige Beschreibung zu den Figuren 1 und 2 Bezug genommen. Nachfolgend wird im Wesentlichen auf die Unterschiede zwischen den Ausführungsbeispielen gemäß den Figuren 1 und 2 sowie 10, 11 und 12 eingegangen.

Der Haltearm 1 ist im Wesentlichen wie der Haltearm 1 gemäß Figur 1 und 2 aufgebaut, allerdings sind an den drei Knick-Gelenken 13, 17, 21 Anzeigeeinheiten 250, 252, 254 angeordnet, die jeweils zwei Displays 260, 261, 262, 263, 264, 265 aufweisen. In Figur 10 sind allein die Displays 260, 262, 264 zu sehen, die Displays 261, 263, 265 sind auf der bezogen auf Figur 10 hinteren Seite der Haltevorrichtung 1 parallel zu den Displays 260, 262, 264 angeordnet.

Die Displays 260, 261, 262, 263, 264, 265 sind rund ausgebildet und mit ihrer Zentralachse koaxial zur Schwenkachse des entsprechenden Gelenks 213, 217, 221 angeordnet.

In Figur 10 ist dargestellt, dass die Displays 260, 262 eine Zeitdauer anzeigen, die das betreffende Gelenk 13, 17 bereits in dieser Position verweilt. Dies ist insbesondere dann hilfreich, wenn ein bestimmter Bewegungsablauf geplant ist. Das Display 264 zeigt gemäß Figur 10 einen Winkelbereich an, in dem das Armsegment 22 geneigt ist. Hierdurch erhält ein Bediener Rückmeldung darüber, wie die Ausrichtung des letzten Armsegments ist, an dem das Anbaugerät aufgenommen wird.

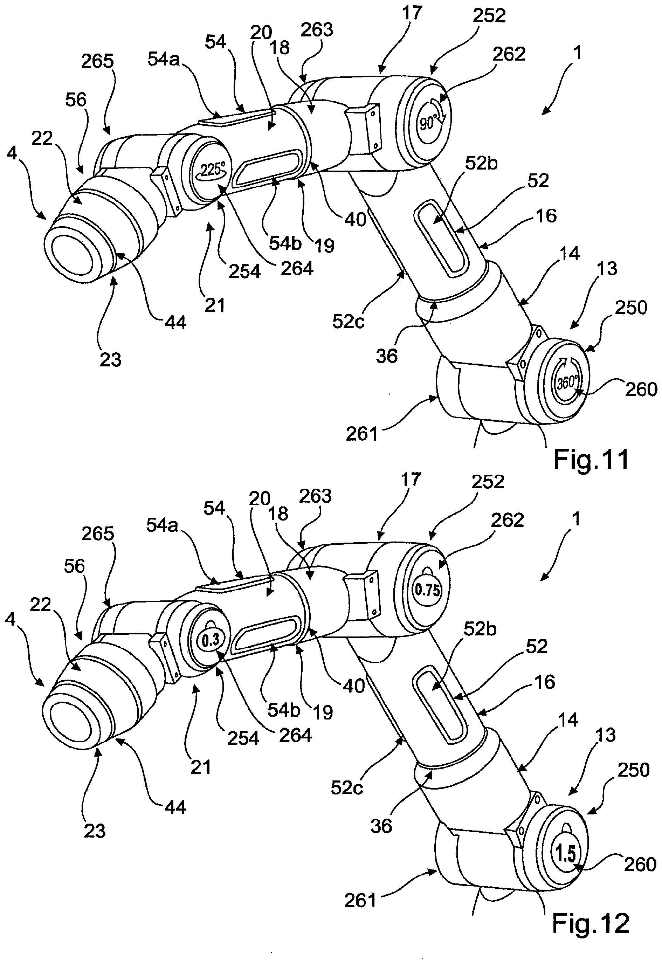

Die Figuren 11 und 12 illustrieren hier gegenüber weitere Ausführungsformen, in denen die Displays 260, 262, 264 genutzt werden, einen vom Freigeben und Arretieren unterschiedlichen Status anzuzeigen.

Gemäß Figur 11 zeigen die Displays 260, 262, 264 an, in welche Richtung und um wie viel Grad ein entsprechendes Gelenk 13, 17, 21 verschwenkt werden darf, um nicht aus einem Arbeitsbereich der Haltevorrichtung 1 heraus zu schwenken. Das Display 264 zeigt nicht nur die Drehrichtung des Gelenks 21 sondern auch die des Gelenks 19 an, was durch den horizontalen Pfeil auf dem Display 264 angezeigt ist. Eine entsprechende Visualisierung ist auch bei den Displays 262, 260 möglich, jedoch an diesem Ausführungsbeispiel nicht gezeigt. In Figur 12 ist eine weitere Visualisierung eines Status der Haltevorrichtung 1 gezeigt. Auf den Displays 260, 262, 264 ist jeweils eingetragen, welches Gewicht auf den einzelnen Gelenken 13, 17, 21 lastet, sodass ein Bediener abschätzen kann, ob die Belastung der Haltevorrichtung 1 noch in einem akzeptablen Bereich ist, und auch, ob unter Umständen eine zu große Belastung auf einem Anbaugerät liegt.

Werden als Anzeigeeinheiten Displays 260, 261, 262, 263, 264, 265 verwendet, sind hier weitere Anzeigen eines Status oder dergleichen denkbar. Die einzelnen Displays sind bevorzugt als berührungsempfindliche Displays ausgebildet und dienen auch zur Eingabe von Profilen an die Haltevorrichtung 1. So ist beispielsweise bevorzugt, dass etwa auf dem Display 260, 261 ein Ein-/Aus-Schalter angezeigt wird, und durch Berühren des Displays 260, 261 die Haltevorrichtung ein- bzw. ausgeschaltet werden kann. Ebenso ist denkbar, dass durch Berührung eines Displays 260, 261, 262, 263, 264, 265 eine aktuelle Pose der Haltevorrichtung 1 gespeichert wird. Weitere Anzeigen sind Übertragung von Datentransfer, Anzeigen von Patientendaten, Patientenbildern, wie Röntgenbildern, CT-/MR-Bilder, Planungsschritten, Zugang zu einer Robotersteuerung eines Anbaugeräts sowie Eingaben von Befehlen für das Einbaugerät, Anzeigen einer Arbeitsumgebung, beispielsweise Verbindung mit weiteren System und dergleichen.

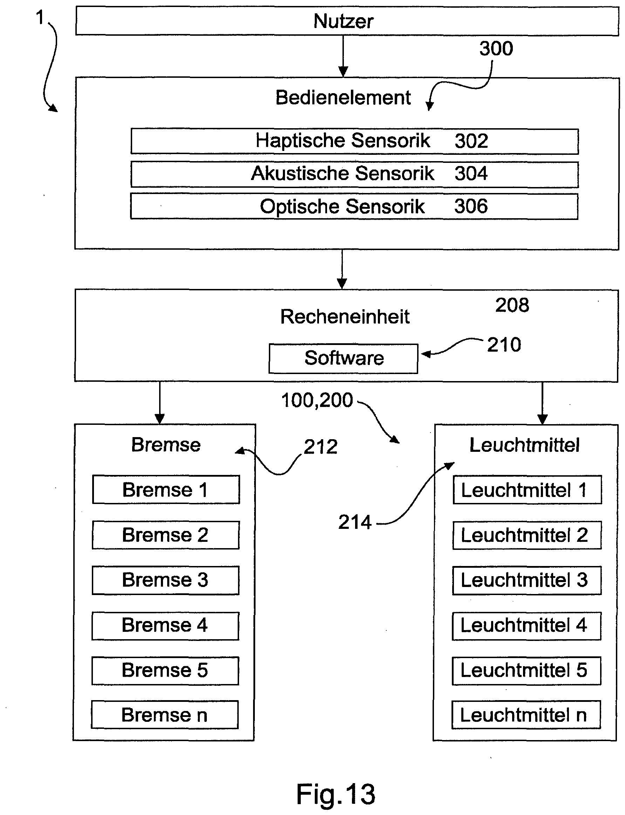

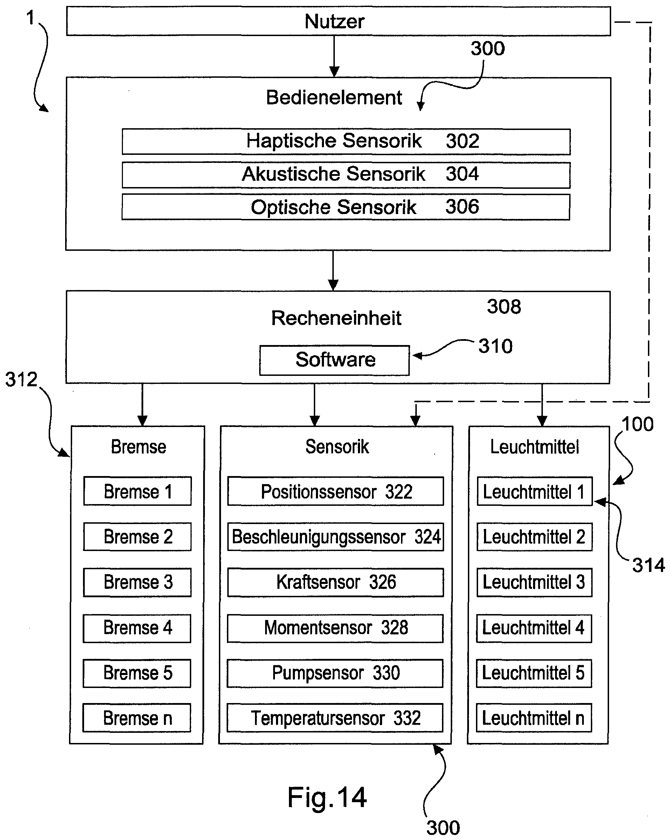

Die Figuren 13 und 14 illustrieren einen grundsätzlichen Aufbau des Systems, umfassend eine Haltevorrichtung 1 sowie einen Nutzer. Die Haltevorrichtung 1 umfasst Bedienelemente 300, die haptische Sensorik 302, akustische Sensorik 304 und optische Sensorik 306 umfassen können. Eine haptische Sensorik ist beispielsweise die Bedieneinrichtung 50, wie vorstehend beschrieben. Intern weist die Haltevorrichtung 1 eine Recheneinheit 308 auf, die einen Softwarebaustein 310 hat. In Gelenken sind Bremsen 312 angeordnet, die in einem RuheZustand geschlossen sind und durch Anliegen einer Spannung geöffnet werden. Dadurch ist die Haltevorrichtung als sogenannte passive Haltevorrichtung ausgebildet und im stromlosen Zustand sind alle Gelenke arretiert. Als Anzeigeeinheiten sind gemäß diesem Ausführungsbeispiel Leuchtmittel 314 vorgesehen, die jeweils einem Gelenk bzw. einer Bremse zugeordnet sind. So ist beispielsweise ein Leuchtmittel 1 der Bremse 1 zugeordnet, Leuchtmittel 2 der Bremse 2 etc. Die einzelnen Leuchtmittel sind vorzugsweise wie in den Figuren 1 bis 7c dargestellt, ausgebildet. Die Recheneinheit ist mit den Bedienelementen gekoppelt und wertet die Bedienung aus, also insbesondere die erfassten haptischen, akustischen oder optischen Signale. Mittels des Softwarebausteins 310 werden diese ausgewertet und entsprechende Bremsen 312 freigegeben und/oder arretiert. Das Freigeben und/oder Arretieren wird dann mit dem entsprechenden Leuchtmittel 314 angezeigt. Gemäß dieser Ausführungsform ist die Haltevorrichtung 1 lediglich in der Lage, das Freigeben und Arretieren von einzelnen Gelenken anzuzeigen, jedoch keinen hiervor verschiedenen Status.

Erfindungsgemäß sind die Anzeigeeinheiten dazu ausgebildet, einen vom Freigeben und Arretieren verschiedenen Status anzuzeigen, wie in Figur 14 dargestellt wird. Dazu weist der Haltearm 1, der grundsätzlich im Aufbau dem aus Figur 13 gleicht, eine zusätzliche Sensorik 320 auf. Die Sensorik 320 umfasst beispielsweise einen oder mehrere Positionssensoren 322 vorzugsweise in jedem Gelenk, einen oder mehrere Beschleunigungssensoren 324, vorzugsweise in jedem Gelenk, einen oder mehrere Kraftsensoren 326, vorzugsweise wenigstens am distalen Ende 4 der Haltevorrichtung 1, einen oder mehrere Momentsensoren 328, vorzugsweise in jedem Gelenk sowie am distalen 4 und am proximalen Ende 2 der Haltevorrichtung 1, wenigstens einen Bumpsensor 330 sowie wenigstens einen Temperatursensor 332. Basierend auf den mittels der Sensorik 320 erfassten Daten, ist die Software 310 dazu eingerichtet, einen Status zu bestimmen und die Anzeigeeinheiten 100 zu veranlassen, diesen Status anzuzeigen, insbesondere mittels der Leuchtmittel 314.

## Patentansprüche

1. Haltevorrichtung (1), insbesondere Haltearm und/oder Stativ, für medizinische Zwecke, insbesondere zum Halten eines Anbaugeräts, insbesondere eines chirurgischen mechatronischen Assistenzsystemen und/oder chirurgischen Instrumenten, mit
einem proximalen Ende (2) zum Befestigen der Haltevorrichtung (1) an einer Basis und einem distalen Ende (4) zum Aufnehmen eines Anbaugeräts (6);
wenigstens einem ersten und einem zweiten Armsegment (12, 14), wobei das erste Armsegment (12) mit einem ersten Gelenk (13) und das zweite Armsegment (14) mit einem zweiten Gelenk (15) verbunden ist, wobei jedes Gelenk (13, 15) freigebbar und arretierbar ist;
einer Bedieneinrichtung (50) zum Freigeben und/oder Arretieren des entsprechenden Gelenks (13, 15) zum Verbringen der Haltevorrichtung (1) in eine gewünschte Pose, wobei die Bedieneinrichtung (50) dazu eingerichtet ist, bei Kontakt zwischen einer Bedienperson und einem der ersten und zweiten Armsegmente (12, 14) das zugeordnete Gelenk (13, 15) freizugeben; und
einer ersten Anzeigeeinheit (34, 100, 200, 250), die an dem ersten Gelenk (13) angeordnet ist und einer zweiten Anzeigeeinheit (36, 100, 200, 252), die an dem zweiten Gelenk (15) angeordnet ist,
**dadurch gekennzeichnet, dass** die erste und/oder zweite Anzeigeeinheit (34, 36, 100, 200, 250, 252) dazu eingerichtet sind, wenigstens einen vom Freigeben und Arretieren des entsprechenden Gelenks (13, 15) verschiedenen Status der Haltevorrichtung (1) und/oder eines Anbaugeräts (6) anzuzeigen.

2. Haltevorrichtung nach Anspruch 1, wobei die Anzeigeeinheiten (34, 36, 100, 200, 250, 252) jeweils wenigstens eine Lichtquelle (102) aufweisen.

3. Haltevorrichtung nach Anspruch 2, wobei die Anzeigeeinheiten (250, 252, 254) wenigstens ein Display (260, 261, 262, 263, 264, 265) zum Anzeigen des Status aufweisen.

4. Haltevorrichtung nach einem der vorstehenden Ansprüche, wobei wenigstens eine Anzeigeeinheit (34, 36, 100) im Wesentlichen ringförmig um eine Schwenkachse des entsprechenden Gelenks (13, 15) ausgebildet ist.

5. Haltevorrichtung (1) nach einem der vorstehenden Ansprüche, wobei der Status ein Betriebsbereitheits-Status der Haltevorrichtung (1) ist.

6. Haltevorrichtung (1) nach einem der vorstehenden Ansprüche, wobei der Status ein Abstand zwischen einem Anbaugerät (6) und einem Rand eines vordefinierten Arbeitsbereichs der Haltevorrichtung (1) ist.

7. Haltevorrichtung (1) nach einem der vorstehenden Ansprüche, wobei der Status ein Abstand zwischen einer aktuellen Pose der Haltevorrichtung (1) und einer vordefinierten Pose der Haltevorrichtung (1) ist.

8. Haltevorrichtung (1) nach Anspruch 7, wobei die Anzeigeeinheiten (34, 36, 100) dazu eingerichtet sind, eine Richtung anzuzeigen, in die wenigstens ein Gelenk (13, 15) zu bewegen ist, um die Haltevorrichtung (1) von der aktuellen Pose in die vordefinierte Pose zu bewegen.

9. Haltevorrichtung (1) nach einem der vorstehenden Ansprüche, wobei der Status ein Arbeitsstatus einer Steuerung der Haltevorrichtung (1) und/oder ein Kommunikationsstatus der Haltevorrichtung (1) mit dem Anbaugerät (6) ist.

10. Haltevorrichtung (1) nach einem der vorstehenden Ansprüche, wobei der Status eine Bewegung der Haltevorrichtung (1) ist.

11. Haltevorrichtung (1) nach einem der vorstehenden Ansprüche, mit einem Kraftsensor zum Messen einer an dem Anbaugerät (6) angreifenden Kraft, wobei der Status die gemessene Kraft ist.

12. Haltevorrichtung (1) nach einem der vorstehenden Ansprüche, wobei der Status eine auf ein Gelenk (13, 15) wirkende Kraft ist.

13. Haltevorrichtung (1) nach einem der vorstehenden Ansprüche, wobei die Anzeigevorrichtungen (34, 36, 100) dazu eingerichtet sind anzuzeigen, ob ein entsprechendes Gelenk (13, 15) freigegeben oder arretiert ist.

14. Verfahren zum Anzeigen wenigstens eines von einem Freigeben und Arretieren eines Gelenks (13, 15) einer Haltevorrichtung (1) nach einem der vorstehenden Ansprüche verschiedenen Status der Haltevorrichtung (1) und/oder eines Anbaugeräts (6), mit den Schritten:
- Erfassen des Status;
- Anzeigen des Status, insbesondere mittels einer im Wesentlichen ringförmig um eine Schwenkachse eines entsprechenden Gelenks ausgebildeten Anzeigeeinheit (34, 36, 100).

## Claims

1. A holding apparatus (1), in particular a holding arm and/or a tripod, for medical purposes, in particular for holding an attachment, in particular a surgical mechatronic assistance system and/or a surgical instrument, comprising
a proximal end (2) for attaching the holding apparatus (1) to a base and a distal end (4) for receiving an add-on device (6);
at least one first and one second arm segment (12, 14), wherein the first arm segment (12) is connected to a first joint (13) and the second arm segment (14) is connected to a second joint (15), wherein each joint (13, 15) is releasable and lockable;
an operating device (50) for releasing and/or locking the respective joint (13, 15) for putting the holding apparatus (1) into a desired pose, wherein the operating device 50 is adapted to release the associated joint (13, 15), upon contact between an operator and one of the first and second arm segments (12, 14); and
a first display unit (34, 100, 200, 250) which is arranged on the first joint (13) and a second display unit (36, 100, 200, 252) which is arranged on the second joint (15),
**characterised in that** the first and/or second display unit (34, 36, 100, 200, 250, 252) is configured to display at least one status of the holding apparatus (1) and/or of an add-on device (6) that is different from the releasing and/or locking of the respective joint (13, 15).

2. The holding apparatus (1) according to claim 1, wherein the display units (34, 36, 100, 200, 250, 252) each have at least one light source (102).

3. The holding apparatus (1) according to claim 2, wherein the display units (250, 252, 254) have at least one display (260, 261, 262, 263, 264, 265) for displaying the status.

4. The holding apparatus (1) according to any one of the preceding claims, wherein at least one display unit (34, 36, 100) is substantially designed as a ring around a pivot axis of the respective joint (13, 15).

5. The holding apparatus (1) according to any one of the preceding claims, wherein the status is an operational readiness status of the holding apparatus (1).

6. The holding apparatus (1) according to any one of the preceding claims, wherein the status is a distance between an add-on device (6) and an edge of a predefined operating area of the holding apparatus (1).

7. The holding apparatus (1) according to any one of the preceding claims, wherein the status is a distance between a current pose of the holding apparatus (1) and a predefined pose of the holding apparatus (1).

8. The holding apparatus (1) according claim 7, wherein the display units (34, 36, 100) are configured to display a direction in which at least one joint (13, 15) is to be moved in order to move the holding apparatus (1) from the current pose into the predefined pose.

9. The holding apparatus (1) according to any one of the preceding claims, wherein the status is an operational status of a controller for the holding apparatus (1) and/or a communication status of the holding apparatus (1) with the add-on device (6).

10. The holding apparatus (1) according to any one of the preceding claims, wherein the status is a movement of the holding apparatus (1).

11. The holding apparatus (1) according to any one of the preceding claims, comprising a force sensor for measuring a force applied to the add-on device (6), wherein the status is the measured force.

12. The holding apparatus (1) according to any of the preceding claims, wherein the status is a force applied to a joint (13, 15).

13. The holding apparatus (1) according to any one of the preceding claims, wherein the display devices (34, 36, 100) are configured to indicate whether a respective joint (13, 15) is released or locked.

14. A method for displaying at least one status of a holding apparatus (1) according to any one of the preceding claims and/or of an add-on device (6), that is different from the releasing and/or locking of a joint (13, 15), said method comprising the steps of:
- detecting the status;
- displaying the status, in particular by means of a display unit (34, 36, 100) which is substantially designed as a ring around a pivot axis of the respective joint.

## Revendications

1. Système (1) de support, notamment bras de support et/ou statif, à des fins médicales, notamment pour supporter un appareil rapporté, notamment un système d'assistance chirurgicale mécatronique et/ou un instrument chirurgical, comprenant
une extrémité (2) proximale pour la fixation du système (1) de support à un socle et une extrémité (4) distale pour la réception d'un appareil (6) rapporté;
au moins un premier et un deuxième segments (12, 14) de bras étant reliés à une première articulation (13) et le deuxième segment (14) de bras à une deuxième articulation (15), chaque articulation (13, 15) pouvant être débloquée et bloquée;
un dispositif (50) de manœuvre pour débloquer et/ou bloquer l'articulation (13, 15) correspondante, afin de mettre le système (1) de support en une position souhaitée, le dispositif (50) de manœuvre étant conçu pour, au contact entre un opérateur et l'un des premier et deuxièmes segments (12, 14) de bras, débloquer l'articulation (13, 15) associée et
une première unité (34, 100, 200, 250) d'affichage, qui est montée sur la première articulation (13), et une deuxième unité (36, 100, 200, 252) d'affichage, qui est montée sur la deuxième articulation (15),
**caractérisé en ce que** la première et/ou la deuxième unités (34, 36, 100, 200, 250, 252) d'affichage sont conçues pour afficher au moins un état, différent du déblocage et du blocage de l'articulation (13, 15) correspondante, du système (1) de support et/ou d'un appareil (6) rapporté.

2. Système de support suivant la revendication 1, dans lequel les unités (34, 36, 100, 200, 250, 252) d'affichage ont chacune au moins une source (102) lumineuse.

3. Système de support suivant la revendication 2, dans lequel les unités (250, 252, 254) d'affichage ont au moins un écran (260, 261, 262, 263, 264, 265) pour l'affichage de l'état.

4. Système de support suivant l'une des revendications précédentes, dans lequel au moins une unité (34, 36, 100) d'affichage est constituée de manière sensiblement annulaire autour d'un axe de pivotement de l'articulation (13, 15) correspondante.

5. Système (1) de support suivant l'une des revendications précédentes, dans lequel l'état est un état de prêt à fonctionner du système (1) de support.

6. Système (1) de support suivant l'une des revendications précédentes, dans lequel l'état est une distance entre un appareil (6) rapporté et un bord d'une partie de travail définie à l'avance du système (1) de support.

7. Système (1) de support suivant l'une des revendications précédentes, dans lequel l'état est une distance entre une position en cours du système (1) de support et une position définie à l'avance du système (1) de support.

8. Système (1) de support suivant la revendication 7, dans lequel les unités (34, 36, 100) d'affichage sont conçues pour indiquer un sens, dans lequel il faut déplacer au moins une articulation (13, 15) pour faire passer le système (1) de support de la position en cours à la position définie à l'avance.

9. Système (1) de support suivant l'une des revendications précédentes, dans lequel l'état est un état de travail d'une commande du système (1) de support et/ou un état de communication du système (1) de support avec l'appareil (6) rapporté.

10. Système (1) de support suivant l'une des revendications précédentes, dans lequel l'état est un mouvement du système (1) de support.

11. Système (1) de support suivant l'une des revendications précédentes, comprenant un capteur de force pour mesurer une force attaquant l'appareil (6) rapporté, l'état étant la force mesurée.

12. Système (1) de support suivant l'une des revendications précédentes, dans lequel l'état est une force s'appliquant à une articulation (13, 15).

13. Système (1) de support suivant l'une des revendications précédentes, dans lequel les unités (34, 36, 100) d'affichage sont conçues pour indiquer si une articulation (13, 15) correspondante est débloquée ou bloquée.

14. Procédé d'affichage d'au moins un état, différent d'un déblocage et d'un blocage d'une articulation (13, 15), d'un système (1) de support suivant l'une des revendications précédentes, du système (1) de support et/ou d'un appareil rapporté, comprenant les stades :
- détection de l'état
- affichage de l'état, notamment au moyen d'une unité (34, 36, 100) d'affichage, constituée de manière sensiblement annulaire autour d'un axe de pivotement d'une articulation correspondante.
